Europäisches Patentamt

**European Patent Office**

Office européen des brevets

(11) Veröffentlichungsnummer: **0 093 277**

A1

(12) **EUROPÄISCHE PATENTANMELDUNG**

(21) Anmeldenummer: 83103449.1

(22) Anmeldetag: 08.04.83

(51) Int. Cl.³: **G 10 K 11/00**
**A 61 B 10/00**

(30) Priorität: 26.04.82 DE 3215561

(43) Veröffentlichungstag der Anmeldung:
09.11.83 Patentblatt 83/45

(84) Benannte Vertragsstaaten:
AT CH DE FR GB LI

(71) Anmelder: SIEMENS AKTIENGESELLSCHAFT
Berlin und München Wittelsbacherplatz 2
D-8000 München 2(DE)

(72) Erfinder: Hetz, Walter
Adam-Kraft-Strasse 17
D-8520 Erlangen(DE)

(54) Ultraschall-Applikator.

(57) Die Erfindung bezieht sich auf einen Ultraschall-Applikator für Ultraschallabtastung, insbesondere von körperinternen Organen od.dgl., mit einem Ultraschallkopf, der an einem Hangriff gehaltert ist. Ziel der Erfindung ist es, einen Ultraschall-Applikator aufzubauen, der sich auch zum intra-operativen Einsatz eignet. Dieses Ziel wird erfindungsgemäß dadurch erreicht, daß der Ultraschallkopf (1) etwa in der Mitte des Schallkopfgehäuses (2) am Handgriff (11) bieg-, schwenk- oder drehbar gehaltert ist.

FIG 1

0093277

SIEMENS AKTIENGESELLSCHAFT          Unser Zeichen
Berlin und München                  VPA 82 P 3740 E

Ultraschall-Applikator

Die Erfindung bezieht sich auf einen Ultraschall-Applikator gemäß den Merkmalen des Oberbegriffs des Patentanspruchs 1.

Es sind bereits Ultraschall-Applikatoren dieser Art vorgeschlagen worden, die jedoch alle nur zur Abtastung von körperinternen Organen von der unversehrten Körperoberfläche her ausgebildet sind. Wünschenswert wäre jedoch ein Ultraschall-Applikator, der beispielsweise auch intra-operativ eingesetzt werden kann.

Aufgabe der Erfindung ist es daher, einen solchen Ultraschall-Applikator zu schaffen, der für den intra-operativen Einsatz geeignet ist.

Die Aufgabe wird erfindungsgemäß mit den kennzeichnenden Merkmalen des Patentanspruchs 1 gelöst.

Ein bieg-, schwenk- oder drehbar gehalterter Ultraschallkopf läßt sich in einfachster Weise in jede beliebige Applikationslage bringen. Bezüglich eines zu untersuchenden körperinternen Organes bedeutet dies, daß der Ultraschall-Applikator beliebig rück-, vorderoder seitenwandig an das abzutastende Organ angelegt werden kann. Der erfindungsgemäße Ultraschall-Applikator eignet sich also insbesondere zum intra-operativen Einsatz.

Weitere Vorteile und Einzelheiten der Erfindung ergeben sich aus der nachfolgenden Beschreibung zweier

Kue 5 Kof / 23.04.1982

- 2 -      VPA 82 P 3740  E

Ausführungsbeispiele anhand der Zeichnung und in Verbindung mit den Unteransprüchen.

Es zeigen:

Figur 1 einen ersten Ultraschall-Applikator gemäß der Erfindung,

Figur 2 einen zweiten Ultraschall-Applikator gemäß der Erfindung,

Figur 3 Applikationsmöglichkeiten für solche Ultraschall-Applikatoren bei intra-operativem Einsatz an z.B. einer Niere.

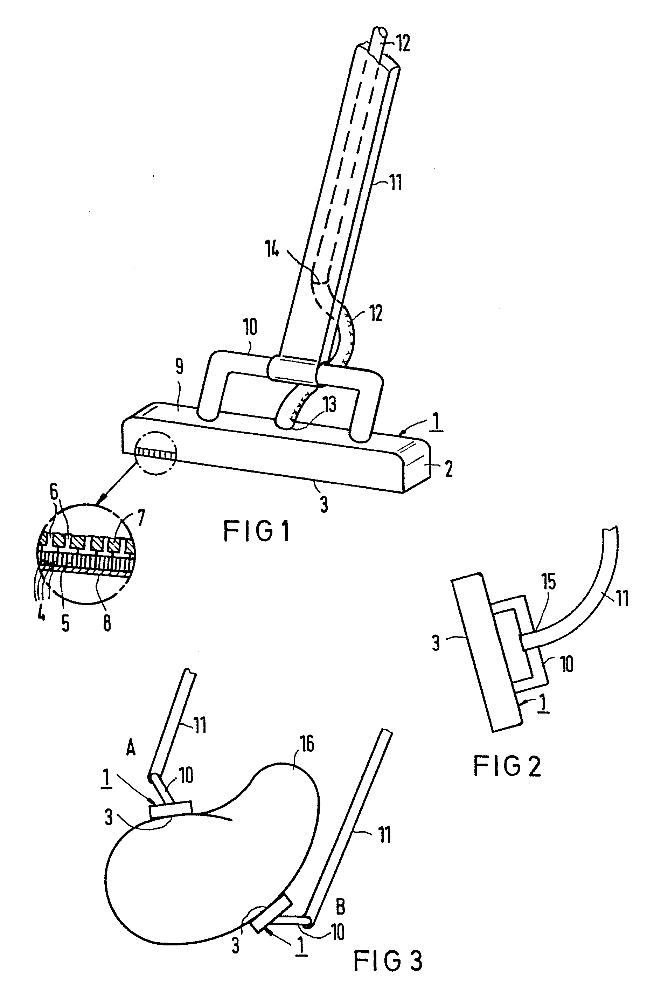

In der Figur 1 ist der Ultraschallkopf ein Ultraschall-Array 1 mit dem Array-Gehäuse 2 und der Applikationsfläche 3. Das Ultraschall-Array 2 umfaßt dabei an der Applikationsfläche 3, wie in der Ausschnittsvergrößerung dargestellt ist, eine Vielzahl von Wandlerelementen 4, die im vorliegenden Ausführungsbeispiel feingeteilt sind. Immer mehrere dieser feingeteilten Wandlerelemente 4, z.B. im vorliegenden Fall insgesamt vier Wandlerelemente, sind elektrisch zu je einer Gruppe 5 (von z.B. insgesamt 48 Gruppen) zusammenkontaktiert. Zur Gruppenkontaktierung dienen Kontaktfahnen 6 eines Kontaktkammes. Mit 7 ist ein Trägerkörper für die Ultraschall-Wandlerelemente 4 bezeichnet. Der Trägerkörper, in den die Kontaktfahnen 6 eingebettet sind, besteht beispielsweise aus Epoxydharz mit eingebrachtem oxidiertem Wolframpulver. Mit 8 ist eine Anpassungsschicht für die Wandlerelemente bezeichnet. Die Anpassungsschicht kann aus Epoxydharz bestehen. Das Grundprinzip der Feinteilung von Wandlerelementen ist z.B. in der US-PS 43 05 014 näher beschrieben.

- 3 -    VPA 82 P 3740 E

Gemäß der Ausführungsform der Figur 1 weist das Ultraschall-Array 1 am Array-Gehäuse 2 an der der Applikationsfläche 3 gegenüberliegenden Gehäusefläche 9 in zentraler Position einen Bügel 10 auf. An diesem Bügel 10 ist ein Handgriff 11 um den Bügel 10 als Dreh- oder Schwenkachse mit vorgebbarer Reibung dreh- oder schwenkbar angelenkt. Mit 12 ist das Signalanschlußkabel für das Ultraschall-Array 1 bezeichnet. Das Signalanschlußkabel 12, das das Array-Gehäuse 2 durch eine Öffnung 13 verläßt, mündet im Handgriff 11 in einer Öffnung 14. Ab dieser Öffnung 14 verläuft das Signalanschlußkabel 12 frei verschiebbar im hohlen Inneren des Handgriffs 11. Dadurch kann das Kabel 12 dem Ultraschall-Array 1 praktisch zugfrei in jede beliebige Schwenk- oder Drehlage folgen.

Die Ausführungsform der Figur 2 zeigt ein Ultraschall-Array 1 mit einem Bügel 10, an dem der Handgriff 11 biegsam gehaltert ist. Dazu kann z.B. der Handgriff 11 lediglich im Bereich der Ankoppelstelle 15 oder aber auch als ganzes Teil aus biegsamem Material, z.B. biegsamem Kunststoff, hergestellt sein.

Der Ultraschall-Applikator der Figur 1 eignet sich insbesondere für den intra-operativen Einsatz, obgleich auch ein subkutaner Einsatz möglich ist. Die Möglichkeit des intra-operativen Einsatzes ist am Beispiel einer Nierenuntersuchung in der Figur 2 dargestellt. Hier ist die durch die Operation freigelegte Niere mit 16 bezeichnet. Das Ultraschall-Array 1 liegt in der Untersuchungsposition A z.B. auf der Vorderwand der Niere 16. In der Untersuchungsposition B liegt das Ultraschall-Array 1 hingegen an der Rückwand der Niere 16. Es ist selbstverständlich, daß derartige Untersuchungen nicht nur an der Niere 16, sondern an jedem beliebigen anderen körper-

- 4 -        VPA 82 P 3740  E

internen Organ oder einem sonstigen inneren Körperteil vorgenommen werden können.

3 Figuren
8 Patentansprüche

Patentansprüche

1. Ultraschall-Applikator für die Ultraschallabtastung, insbesondere von körperinternen Organen od.dgl., mit einem Ultraschallkopf, der an einem Handgriff gehaltert ist, d a d u r c h   g e k e n n z e i c h n e t , daß der Ultraschallkopf (1) etwa in der Mitte des Schallkopfgehäuses (2) am Handgriff (11) bieg-, schwenk- oder drehbar gehaltert ist.

2. Ultraschall-Applikator nach Anspruch 1, d a - d u r c h   g e k e n n z e i c h n e t , daß der Ultraschallkopf (1) und der Handgriff (11) wenigstens an der Ankoppelstelle biegsam verbunden sind.

3. Ultraschall-Applikator nach Anspruch 1 oder 2, d a d u r c h   g e k e n n z e i c h n e t , daß der gesamte Handgriff (11), gegebenenfalls sogar wenigstens teilweise der Ultraschallkopf (1), aus biegsamem Material hergestellt ist.

4. Ultraschall-Applikator nach Anspruch 1, d a - d u r c h   g e k e n n z e i c h n e t , daß der Ultraschallkopf (1) mittels Schwenk- oder Drehgelenk (10), das sich am einen Ende des Schallkopfgehäuses (2) befindet, am Handgriff (11) schwenk- oder drehbar gehaltert ist.

5. Ultraschall-Applikator nach einem der Ansprüche 1 bis 4, d a d u r c h   g e k e n n z e i c h n e t , daß der Ultraschallkopf (1) am Schallkopfgehäuse (2) in zentraler Position einen Bügel (10) aufweist, an dem der Handgriff (11) bieg-, dreh- oder schwenkbar angelenkt ist.

6. Ultraschall-Applikator nach Anspruch 4 und 5, d a d u r c h   g e k e n n z e i c h n e t , daß der Bügel (10) selbst eine Dreh- oder Schwenkachse für den Handgriff (11) bildet.

7. Ultraschall-Applikator nach Anspruch 5 oder 6, d a d u r c h   g e k e n n z e i c h n e t , daß der Bügel (10) unter vorgebbarem Winkel schräg zur Applikationsfläche (3) des Ultraschallkopfes verläuft.

8. Ultraschall-Applikator nach einem der Ansprüche 1 bis 7, d a d u r c h   g e k e n n z e i c h n e t , daß der Ultraschallkopf (1) ein Ultraschall-Array ist.

0093277

FIG 1

FIG 2

FIG 3

**Europäisches Patentamt**

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | EP-A-0 039 851 (OLYMPUS OPTICAL CO.) <br> * Seite 3, Zeile 21 - Seite 5, Zeile 5; Figuren 2-6 * | 1,4-6 | G 10 K 11/00 <br> A 61 B 10/00 |
| | --- | | |
| P | DE-A-3 121 512 (S.R.I.) <br><br> * Seite 12, Zeile 25 - Seite 13, Zeile 6; Seite 14, Zeilen 18-31; Seite 15, Zeile 30 - Seite 16, Zeile 26; Figuren 1,3,4 * | 1,2,4, 8 | |
| | --- | | |
| Y | DE-B-2 826 828 (SIEMENS) <br> * Spalte 4, Zeile 57 - Spalte 5, Zeile 12; Figur 1 * | 1,6,8 | |
| | --- | | |
| A | DE-A-2 950 203 (OLYMPUS OPTICAL CO.) | 1,6,8 | RECHERCHIERTE SACHGEBIETE (Int. Cl. 3) |
| | --- | | |
| A | EP-A-0 039 045 (OLYMPUS OPTICAL CO.) | | G 10 K 11 <br> A 61 B 10 <br> A 61 H 23 <br> G 01 N 29 |
| | --- | | |
| A | US-A-1 416 281 (B. GILIBERTI) | | |
| | ----- | | |

Der vorliegende Recherchenbericht wurde für alle Patentansprüche erstellt.

| Recherchenort <br> DEN HAAG | Abschlußdatum der Recherche <br> 16-08-1983 | Prüfer <br> HAASBROEK J.N. |
|---|---|---|